(19) Europäisches Patentamt / European Patent Office / Office européen des brevets

(11) **EP 2 144 034 A1**

(12) **EUROPEAN PATENT APPLICATION**

(43) Date of publication:
**13.01.2010 Bulletin 2010/02**

(51) Int Cl.:
**G01B 9/02** (2006.01)          **G01B 11/06** (2006.01)
**A61B 3/10** (2006.01)

(21) Application number: **09163477.4**

(22) Date of filing: **23.06.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **09.07.2008 JP 2008179311**

(71) Applicant: **Canon Kabushiki Kaisha**
**Tokyo 146-8501 (JP)**

(72) Inventor: **Suehira, Nobuhito**
**Tokyo Tokyo 146-8501 (JP)**

(74) Representative: **TBK-Patent**
**Bavariaring 4-6**
**80336 München (DE)**

(54) **Multilayer structure measuring method and multilayer structure measuring apparatus**

(57)    A tomographic image measuring method according to the present invention includes a first step of calculating information corresponding to an optical distance of each layer thickness from a wave-number spectrum, a second step of separating and extracting information of each layer from the information corresponding to the optical distance of each layer thickness, a third step of reconverting information of each layer into a wave-number spectrum, respectively, a fourth step of obtaining a interference wave number from the result of the third step, a fifth step of calculating an order of interference from the interference wave number and the optical distance of each layer; and a sixth step of calculating the optical distance of each layer by making use of the fact that the order of interference is an integer. Thus, when discrete Fourier transform is used, the measuring precision of layers is improved.

*Fig. 3A*

START SIGNAL PROCESSING

S1 — ACQUIRE WAVE NUMBER SPECTRUM

S2 — PERFORM FOURIER TRANSFORM

S3 — EXTRACT ONE PEAK

S4 — PERFORM INVERSE FOURIER TRANSFORM

S5 — PROCESSING OF ALL PEAKS COMPLEATED ? — NO / YES

S6 — SPECIFY INTERFERENCE WAVE NUMBER (WAVELENGTH)

S7 — CALCULATE ORDER OF INTERFERENCE m

S8 — CALCULATE OPTICAL DISTANCE OF LAYER THICKNESS BY USING m APPROXIMATED TO AN INTEGER

S9 — MEASURING IN ALL REGIONS COMPLETED ? — NO / YES

END

EP 2 144 034 A1

**Description**

BACKGROUND OF THE INVENTION

Field of the Invention

The present invention relates to a technique that measures a multilayer structure by using optical-coherence optical system.

Description of the Related Art

Today, as a multilayer structure measuring apparatus (optical coherence apparatus) that measures a multilayer structure by using an optical-coherence optical system, there are used a variety of kinds of such ones. For example, there are a reflection spectrum film-thickness measuring apparatus that measures the thickness of a thin film arranged on a surface of a semiconductor or glass, and an optical-coherence tomographic imaging apparatus (Optical Coherence Tomography: OCT, hereinafter referred to as an OCT apparatus) that images the tomographic structure of a light scattering medium such as a living body or the like. These are the same in using light interference, but the names and/or constructions of the apparatuses are different according to fields of use and objects to be observed. Here, an object having a film structure on a surface, such as a resist of a semiconductor or the like is called a film, and an object having a structure in its interior, such as a retina of an eye, is called a sectioned layer or simply a layer.

In the above-mentioned apparatuses, by irradiating measurement light onto an object to be observed and analyzing the light reflected therefrom, it is possible to measure the structure of films, layers or the like of the object. For its light source (sometimes referred to as a broad-band light source, a white light source, a low-coherence light source, etc., depending upon fields of use), an appropriate wavelength and bandwidth should be selected according to the structure and characteristic of the object to be observed. In addition, there are a method of observing the interference of the reflected light at each level or layer of the object to be observed by using only the reflected light from the object to be observed, and a method of observing the interference of combined light which is composed of light reflected from a reference mirror and light reflected from the object to be observed. These reflected lights are analyzed by using a spectroscope and imaged as optical spectrum by means of a line sensor or the like.

The spectral data thus imaged is analyzed by using Fourier transforms, etc., so that the structure of films or layers can be obtained. Further, a two-dimensional tomographic image can be obtained by scanning measurement light on a sample in a two-dimensional manner.

Japanese patent application laid-open No. H11-325849 discloses an OCT used for medical application. Here, the position of a reference mirror is changed three times in a discontinuous manner, so that a spectrum is obtained at each position. Then, a tomographic image is obtained by performing calculations with the use of these pieces of data. It is said that with such a technique, deterioration resolution can be prevented.

SUMMARY OF THE INVENTION

In Japanese patent application laid-open No. H11-325849, the position of the reference mirror is changed a plurality of times for one measurement. Such a scheme not only takes much time for measurements but also requires precise position control of the reference mirror. On the other hand, in OCT used in the medical field, there is a method of measuring sectioned layers by means of a spectroscope with a reference mirror being fixed and by Fourier transforming spectral data from the spectroscope. The spectral data at this time becomes a discrete Fourier transform because the number of pixels of a line sensor is limited. As a result, the values of the layers can only be expressed as discrete values. Therefore, an improvement in the measuring precision of the layers has been required.

Accordingly, the present invention has been made in view of the above-mentioned problems, and provides a tomographic image in a more accurate manner.

A first aspect of the present invention is a multilayer structure measuring method as specified in the claims 1 through 12.

A second aspect of the present invention is a multilayer structure measuring apparatus as specified in the claims 13 and 14.

According to the present invention, in a multilayer structure measuring apparatus, it is possible to obtain a tomographic image of which measuring precision is improved.

Further features of the present invention will become apparent from the following description of exemplary embodiments with reference to the attached drawings.

BRIEF DESCRIPTION OF THE DRAWINGS

Fig. 1 is a view for explaining an optical system of an OCT apparatus in a first embodiment of the present invention.
Fig. 2 is a view for explaining a layer structure in the first embodiment of the present invention.
Fig. 3A is a flow chart illustrating a flow of signal processing in the first embodiment of the present invention.
Fig. 3B is a schematic diagram illustrating a spectral waveform of measuring light in the first embodiment of the present invention.
Fig. 3C is a schematic diagram illustrating the result of a discrete Fourier transform in the first embodiment of the present invention.
Fig. 3D is a schematic diagram illustrating a spectral waveform as a result of an inverse Fourier transform of a spectral signal after having been band-pass filtered in the first embodiment of the present invention.
Fig. 4 is a view for explaining an optical system of an OCT apparatus with the use of a reference mirror in a second embodiment of the present invention.
Fig. 5 is a view for explaining a layer structure in the second embodiment of the present invention.
Fig. 6A is a schematic diagram illustrating a spectral waveform of measuring light in the second embodiment of the present invention.
Fig. 6B is a schematic diagram illustrating the result of a discrete Fourier transform in the second embodiment of the present invention.
Fig. 6C is a schematic diagram illustrating a spectral waveform as a result of an inverse Fourier transform of a spectral signal after having been band-pass filtered in the second embodiment of the present invention.
Fig. 7 is a view for explaining an optical system of an ophthalmic OCT apparatus in a third embodiment of the present invention.

DESCRIPTION OF THE EMBODIMENTS

An optical coherence apparatus (multilayer structure measuring apparatus) according to the present invention irradiates measurement light onto an object to be inspected (an object to be observed) through a sample arm (measurement light path), and guides return light of this measuring light from the object to be inspected to a detection position. The return light is reflected light or scattered light that contains information on an interface in a direction of the light irradiated to the object to be inspected, etc. In addition, a tomographic image of the object to be inspected is taken by using a spectroscope that detects the wavelength spectrum of the return light guided to the detection position, and an analysis unit that analyzes this spectrum.

In order to solve the problems as described above, the present invention constructs the analysis unit in the multilayer structure measuring apparatus in such a manner that it can measure an optical distance of each level or layer of a multilayer structure according to first through sixth steps, as will be described below.

In the first step, information corresponding to the optical distance is calculated from a wave number spectrum of the return light by means of Fourier transform or the like. The optical distance nd of each layer can be obtained from the position of each corresponding peak. In the second step, information corresponding to the optical distance of each layer of the multilayer structure is separated and extracted from information corresponding to the optical distance. In the third step, the information corresponding to the optical distance of each layer is again converted into a wave number spectrum by inverse Fourier transform or the like. In the fourth step, a wave number (or wavelength) at which interference (constructive or destructive interference) occurs is obtained from the wave number spectrum. In the fifth step, the order of interference is calculated from the wave number (wavelength) thus obtained and the optical distance of each layer. In the sixth step, the order of interference is approximated (corrected) to an integer by using the fact that an order of interference theoretically becomes an integer, and the optical distance of each layer is calculated from the integer thus approximated and the wave number (wavelength) at which the interference occurs.

With the above-mentioned construction, it is possible to obtain a tomographic image of improved measuring precision.

Now, reference will be made to specific embodiments of the present invention.

[First Embodiment]

In a first embodiment of the present invention, reference will be made to an optical coherence apparatus to which the present invention is applied, while using the accompanying drawings.

<Construction of an Optical System>

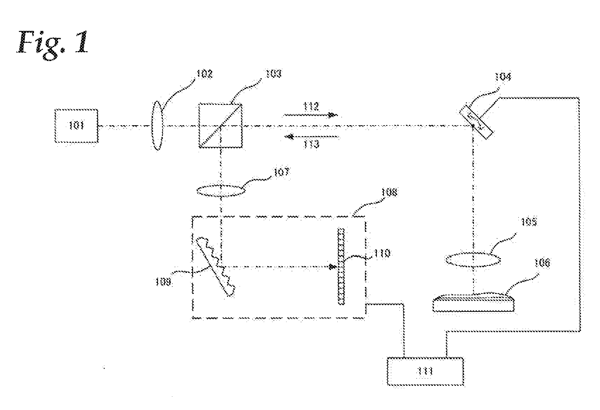

First of all, the construction of the optical coherence apparatus will be roughly described while referring to Fig. 1. Measurement light emitted from a light source 101 arrives at a sample or specimen 106 such as a semiconductor, which is an object to be observed, through a lens 102, a beam splitter 103, an XY scanner 104, and an object lens 105. A transparent film is disposed on a surface of the sample, and the light reflected by the surface of the sample and an interface between the film and the sample arrives at a spectroscope 108 through the object lens 105, the XY scanner 104, the beam splitter 103, and an imaging lens 107.

For the light source 101, a halogen lamp or the like is used which has a wavelength of 400 ~ 800 nm, for instance. Here, for the spectroscope 108, a diffraction grating type spectroscope is used. In this case, the spectroscope 108 is composed of a diffraction grating 109, an image pickup element 110, and so on. The light spectroscoped by the diffraction grating 109 is acquired as spectral data of the wavelength thereof by the image pickup element 110 in the spectroscope 108. The image pickup element 110 is a CCD type line sensor, etc. Here, the merits or advantages of adopting the diffraction grating type spectroscope include the abundance in kinds thereof, substantially constant dispersion in a region of wavelengths used, and so on. On the other hand, the demerits or disadvantages thereof include low transmittance, a lot of stray light, the occurrence of duplication or overlap of the orders of interference, much polarization of the emitted light, and so on. In addition, a prism may be adopted for the spectroscope 108.

The spectral data imaged by the image pickup element 110 is analyzed by a computer 111. Of course, the computer 111 has the functions of not only analyzing the spectral data but also storing it, displaying images, and issuing a command of measurement. In addition, cross-sectional images of the sample can be obtained by raster-scanning the measurement light with respect to the sample in a direction perpendicular to the optical axis thereof by means of the XY scanner 114 under the control of the computer 111. The computer 111 is composed of a CPU, a memory and so on, and achieves the above-mentioned functions by executing a program by means of the CPU. However, part or all of the above-mentioned respective functions can be achieved by hardware.

<Condition of Interference>

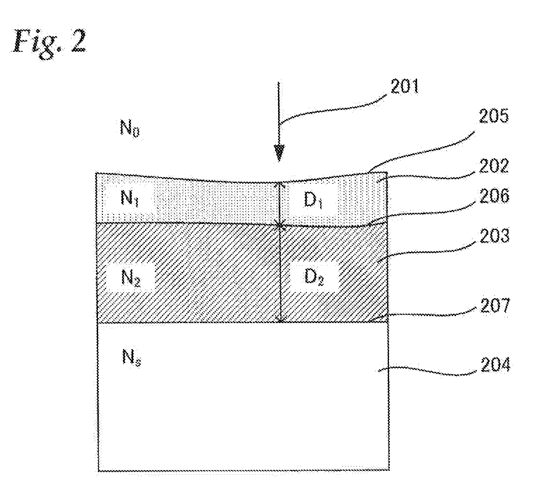

Reference will be made to a condition of interference of the multilayer structure by using Fig. 2. Here, the influence of multiple reflections is not taken into consideration for the sake of simplicity.

The sample or specimen of the multilayer structure is of a construction having a second film 203 of a refractive index $N_2$ and a first film 202 of a refractive index $N_1$ arranged on a substrate 204 of a refractive index $N_s$. It is assumed that this sample is disposed in a medium of a refractive index $N_0$. Respective boundaries are a surface 205, a first interface 206, and a second interface 207. A spatial distance between the surface 205 and the first interface 206 is denoted by $D_1$, and a spatial distance between the first interface 206 and the second interface 207 is denoted by $D_2$. Here, it is assumed that the refractive indexes of adjacent mediums being in contact with each other are different from each other.

Here, when light enters from a medium of a low refractive index to a medium of a high refractive index, the phase of the light reflected on an interface therebetween is not changed. However, when light enters from a medium of a higher refractive index to a medium of a lower refractive index, the phase of the light reflected on an interface therebetween is changed by n. Here, consideration is given that measurements are made on samples having refractive indexes $N_0$, $N_1$, $N_2$, $N_s$ which satisfy a relation of $N_0 < N_1 < N_2 < N_s$. For example, these samples are air of a refractive index $N_0$ ($N_0$ = 1), a resist of a refractive index $N_1$ ($N_1$ = 1.5), silicon nitride of a refractive index $N_2$ ($N_2$ = 2.0), and a silicon substrate of a refractive index $N_s$ ($N_s$ = 3.5). Under this condition, the phase of measurement light 201 is not changed before and after the reflection thereof.

Accordingly, a condition under which lights reflected on interfaces of a certain film at its opposite sides interfere or cohere with each other is denoted, as a constructive condition, by the following expression 1 with the use of a refractive index n of the film, a spatial distance d between the interfaces, an integer (order of interference) m, and a wave number k.

$$k_m = \frac{m}{2nd}$$

In addition, a destructive condition is denoted by the following expression 2.

$$k_{m+0.5} = (m + \frac{1}{2}) \frac{1}{2nd}$$

Of course, the construction of a film is various, and hence should be taken into consideration in the case of a combination of refractive indexes in which there is a change in the phase of light before and after the reflection thereof. As a result, the constructive condition and the destructive condition can be reversed with the above-mentioned expressions 1 and 2.

<Signal Processing>

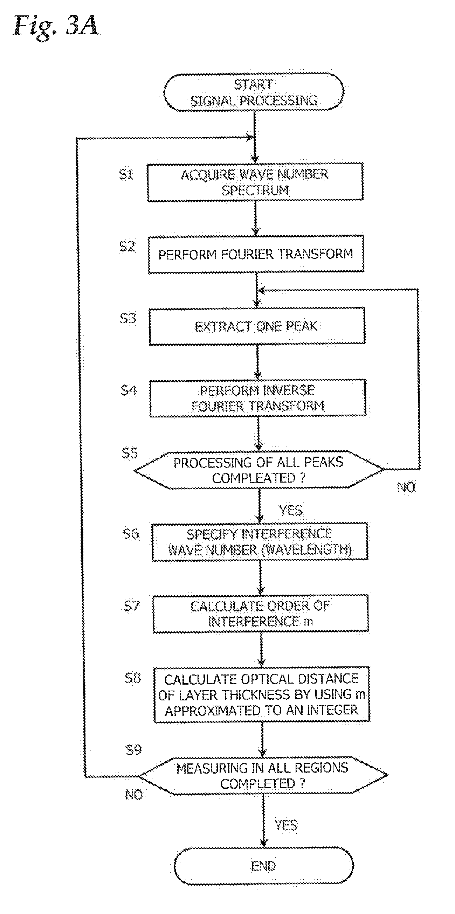

The signal processing steps of the present invention will be described while using Figs. 3A through 3D. Here, an explanation will be made by taking, as an example, a case where the films of a construction as illustrated in Fig. 2 is measured. Here, the following respective steps are executed by the computer 111.

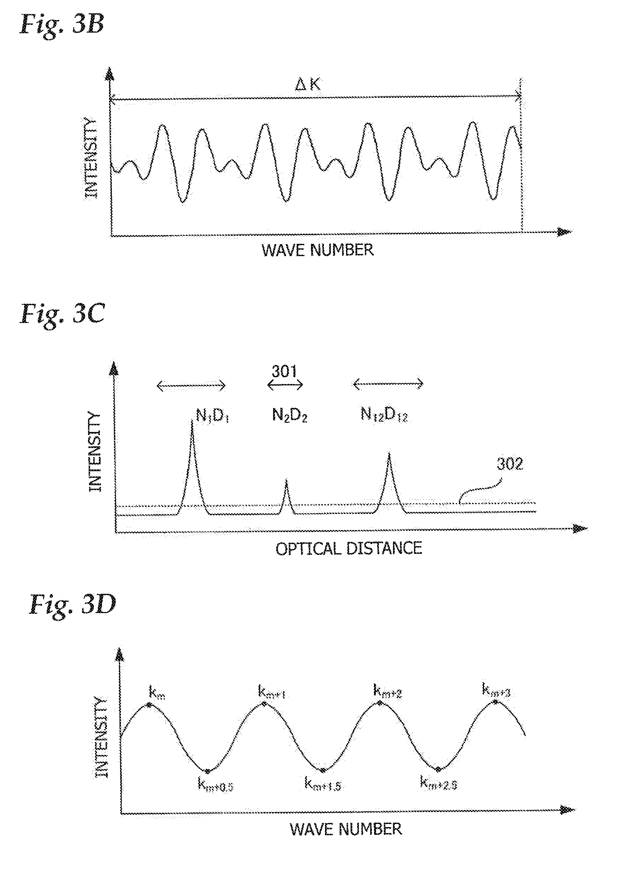

In a step of S1, spectral data from the spectroscope 108 is acquired. The data at this time is diagrammatically illustrated in Fig. 3B. In Fig. 3B, the axis of ordinate denotes intensity or magnitude, and the axis of abscissa denotes wave number. Here, the spectrum of an ordinary spectroscope is often denoted by the intensity with respect to the wavelength thereof. Here, however, a wave number spectrum is used, and thus it is necessary to convert a wavelength into a corresponding wave number that is the reciprocal thereof. Further, the wavelength spectrum is equal interval data with respect to the wavelength, so when converted into a corresponding wave number, which is the reciprocal of the wavelength, the data after conversion has non-equal intervals with respect to the wave number. Therefore, it is necessary to make the thus converted data at equal intervals by means of interpolation processing or the like.

In a step of S2 (corresponding to a first step of the present invention), the spectral data is subjected to Fourier transform. Here, the number of samplings of the line sensor is limited, and so discrete Fourier transform is carried out. In general, a value of 512, 1024, 2048 or the like is selected as the number N of samplings (the number of pixels of the line sensor). The result at this time is diagrammatically illustrated in Fig. 3C. The axis of abscissa in the case of the discrete Fourier transform denotes values proportional to optical distances (i.e., information corresponding to the optical distances). The optical distances can be calculated from these values, respectively. For the sake of simplicity of the explanation, the results obtained by the Fourier transform are hereinafter referred to as optical distances, though the results are values proportional to the optical distances. The axis of abscissa for the optical distances is divided by the number of samplings of the line sensor.

Here, peaks of $N_1 D_1$, $N_2 D_2$ and $N_{12} D_{12}$ are measured, as illustrated in Fig. 3C. Of course, these peak values can be used as the optical distances of the respective films. However, in the discrete Fourier transform, the results of the measurements become discrete values. That is, the results of the measurements of the optical distances are denoted as shown in the following expression 3 by using an integer i and a bandwidth $\Delta K$ of the spectroscope illustrated in Fig. 3B. Here, i is an integer that satisfies a relation of $0 \leqq i \leqq N/2$. In addition, $\lambda_{max}$ and $\lambda_{min}$ are a maximum detection wavelength and a minimum detection wavelength, respectively, of the spectroscope.

$$nd = \frac{i}{2\Delta K}$$

The measurement resolution $\delta(nd)$ of the film thickness, being a minimum possible interval for the optical distances that can be taken as a measurement result, is denoted as shown in the following expression 4. Here, this expression also denotes a minimum distance $nd_{min}$ that can be measured.

$$\delta(nd) = \frac{1}{2\Delta K}$$

In addition, a maximum distance $nd_{max}$ that can be measured is obtained as shown in the following expression 5 by substituting N/2 for i of the above expression 3. This is decided by a sampling theorem.

$$nd_{\max} = \frac{N}{4\Delta K}$$

However, in case where interference occurs at opposite ends of an i-th layer and a t-th layer of the multilayer structure, an optical distance $N_{it}D_{it}$ of the i-th through t-th layers (i.e., an optical distance between the i-th layer and the t-th layer) is the sum of an optical distance $N_{it-1}D_{it-1}$ of the i-th through (t-1)-th layers (i.e., an optical distance between the i-th layer and a (t-1)-th layer) and an optical distance $N_tD_t$ of the t-th layer (i.e., an optical distance between the (t-1)-th layer and the t-th layer), as shown in the following expression 6. If this is repeated in succession, the optical distance $N_{it}D_{it}$ of the i-th through t-th layers (as a whole) coincides with the sum of the optical distances of respective layers of the i-th layer through the t-th layer.

$$N_{it}D_{it} = N_{it-1}D_{it-1} + N_tD_t$$

Of course, these values come out as peaks after the Fourier transform. The number of peaks when there are n layers is the number of combinations when selecting two layers from (n+1) layers. For example, as illustrated in Fig. 3C, three peaks are obtained from a film of two-layer construction as illustrated in Fig. 2.

In a step of S3 (corresponding to a second step of the present invention), an optical distance corresponding to one peak is selected by applying a filter to the result of the Fourier transform. A band-pass filter can be used as such a filter. In this regard, the band here has a unit corresponding to length expressed in terms of a physical quantity. When considering it in terms of digital data, processing is the same without regard to unit, and so it is expediently called as such. Further, a bandwidth is a value that is obtained by multiplying the number of pixels by a conversion coefficient for the purpose of digital processing, and here, discussion can be sometimes made by calling the number of pixels a bandwidth. Accordingly, a bandwidth used herein is different from the width of a general frequency or a general wavelength. A bandwidth 301 of the filter is set in accordance with each of the peaks of $N_1D_1$, $N_2D_2$ and $N_{12}D_{12}$, as illustrated in Fig. 3C. Incidentally, these optical distances are stored in a memory or the like so that they can be read out again in a step of S7.

Bandwidths (ranges of extraction) which are set in the filter can be made, for example, ranges in which peaks are equal to or higher than a threshold 302. In addition, it is desirable to set the bandwidths so that the respective regions should not overlap with each other. Also, the wider a bandwidth, the more components different from a desired optical distance are contained, so each bandwidth should be a necessary minimum. For example, a width twice that width which contacts the threshold is set as a bandwidth. Of course, it is desirable to make the bandwidths variable so as to cope with the case where the intensity is as small as the peak of $N_2D_2$. When a rough layer construction is known, the bandwidths can be fixed.

In a step of S4 (corresponding to a third step of the present invention), the signal after having been filtered is subjected to inverse discrete Fourier transform. The waveform of the signal thus obtained is diagrammatically illustrated in Fig. 3D. There is one peak for an optical distance, thus resulting in a substantially sine wave corresponding to the result of a single layer. That is, a waveform is obtained in which a maximum value and a minimum value are repeated.

In a step of S5, it is determined whether a necessary number of peaks have been subjected to inverse Fourier transform. If not yet, the data of the following peak is subjected to inverse Fourier transform. In this example, three inverse Fourier transforms are performed.

In a step of S6 (corresponding to a fourth step of the present invention), a wave number corresponding to a maximum value or a minimum value of the sine wave is extracted. Here, as illustrated in Fig. 3D, four wave numbers including $k_m$, which correspond to the constructive condition, and three wave numbers including $k_{m+0.5}$, which correspond to the destructive condition, are obtained.

In a step of S7 (corresponding to a fifth step of the present invention), the order of interference is calculated. Specifically, the order of interference m in the interference condition is calculated by substituting the optical distances of the peaks extracted in the step of S3 and the wave numbers of the maximum values extracted in the step of S6 in an expression of a constructive condition (here, expression 1). A ratio of an optical path difference between two reflection waves (twice the optical distance of a layer thickness) to a wave number (corresponding wavelength) which takes a maximum in Fig. 3D is calculated. For the wave number at this time, the smallest one may be selected from among a plurality of maximum values, or a wave number at a peak at which the waveform is stable. Here, note that m is obtained by making use of the wave number at the maximum value and the constructive condition expression, but it can also be obtained by making use of the wave number at the minimum value and the destructive condition expression.

However, the optical distance of each level or layer used in the step of S7 can be calculated from the result in the step of S6. In other words, it can be calculated by using wave numbers $k_m$ and $k_{m+l}$ at two peaks in Fig. 3D, where 1 denotes 1-th from m in Fig. 3D. By removing m from two equations which are obtained by substituting $k_m$ and $k_{m+1}$ in the above-mentioned expression 1, respectively, the optical distance of each layer can be obtained, as shown in the following expression 7. In this manner, the optical distances of respective layers can be calculated by obtaining a plurality of wave numbers that take peaks, from a wave-number spectrum after having been subjected to inverse Fourier transform, and these values can also be used in the step of S7.

$$nd = \frac{l}{2(k_m - k_{m+l})}$$

In general, when a multilayer structure is analyzed, the refractive index of each level or layer is known, and hence a decision can be beforehand made as to whether to use expression 1 or expression 2 in the step of S6. However, in case where the refractive indexes of the respective layers are not known, it is also unknown which expression should be used. In such a case, m is calculated by using expression 1 and expression 2, respectively. Thereafter, optical distances $n_d$ are calculated in $k_{m+1}$, $k_{m+2}$, ⋯, respectively. By selecting the one which provides a minimum error of $n_d$, m can be decided. In addition, with this, it is possible to know the magnitude correlation between the refractive indexes of the respective layers.

In a step of S8 (corresponding to a sixth step of the present invention), the optical distance is recalculated by rounding off m obtained in the step of S7 to an integer $M_m$, and by using the constructive condition or the destructive condition again. Intrinsically, in the constructive interference condition, the optical path difference becomes an integral multiple of the wavelength. In case where m obtained in the step of S7 does not become an integer, the reason for this is due to an error. Accordingly, by approximating (correcting) the value of m thus obtained to the nearest integer $M_m$, such an error contained in m can be excluded. When the constructive condition is used in the step of S7, the optical distance is calculated by using the following expression 8.

$$nd = \frac{M_m}{2k_m} = \frac{M_m \lambda_m}{2}$$

On the other hand, an optical path difference should be a half integral multiple of a wavelength in a destructive condition. In case where a destructive condition is used in the step of S7, the optical distance is calculated by the following expression

$$nd = (M_m + \frac{1}{2})\frac{1}{2k_{m+0.5}} = (M_m + \frac{1}{2})\frac{\lambda_{m+0.5}}{2}$$

In a step of S9, it is determined whether the measurements have been completed in a desired region. When not yet completed, the measurements are carried out in the following region. If a desired number of measurements have been completed by repeating these steps, a tomographic image corresponding to that of Fig. 2 can be obtained.

Here, the direction of depth of the tomographic image is delimited by the number of samplings N. In the present method, a number of measurements equal to or less than the number of samplings are carried out. Accordingly, it is necessary to construct a tomographic image that reflects such a measurement or calculation result. In addition, even when no new tomographic image is constructed, the measured values can be displayed accessorily.

<Examples of Numerical Values>

Here, a simple description will be given by using numerical values. Let us assume that measurements are made so as to check or inspect whether samples prepared meet a designed construction. That is, this is a condition in which samples do not deviate significantly from design values.

A measurement system is assumed to use the entire spectrum of 400 nm ~ 800 nm of the halogen lamp. At this time, a minimum distance $nd_{min}$ or a measurement resolution that can be measured is 400 nm (0.4 $\mu$m), as calculated from the above-mentioned expression 4. Here, the bandwidth $\Delta K$ of the spectroscope 108 is 1.25 x $10^6$ [$m^{-1}$] (=1/400 nm - 1/800 nm). In addition, in case where the number of samplings N of the spectroscope 108 is 1, 024 (i.e., N = 1024), a maximum distance $nd_{max}$ that can be measured is 205 $\mu$m, as calculated from the above-mentioned expression 5.

Here, it is assumed that the sample has a spatial distance $D_1$ of about 5 $\mu$m ($D_1$ = 5 $\mu$m) and a spatial distance $D_2$ of about 10 $\mu$m ($D_2$ = 10 $\mu$m), and that a layer of $N_1$ = 1.5 and a layer of $N_2$ = 2 are formed on a Si substrate of $N_s$ = 3.5. The optical distances are approximately as follows: $N_1D_1$ = 7.5 $\mu$m, $N_2D_2$ = 20 $\mu$m, and $N_{12}D_{12}$ = 27.5 $\mu$m, respectively, in the vicinity of which there will be generated peaks as a result of Fourier transform (S2 in Fig. 3A).

When the data of a sampling number N (here 1,024) is subjected to discrete Fourier transform, the axis of abscissa is divided into N pieces. Here, the result thus obtained becomes symmetric with respect to N/2 (= 512), and hence, the following discussion will be made about one side alone. First of all, the measurement resolution is 0.4 $\mu$m, and so in the step of S2, peaks will occur in the vicinity of 19th pixel ($\approx$ 7.5/0.4), 50th pixel ($\approx$ 20/0.4), and 69thpixel ($\approx$ 27.5/0.4), respectively, on the axis of abscissas which is divided into 512. Since the intervals between the adjacent peaks are apart from each other, these peaks can be separated from each other by setting the band-pass filter in three bands in the step of S3. In the step of S4, inverse discrete Fourier transform is performed. First, when the optical distance of the layer thickness is 7 $\mu$m, a peak of m = 19 ($\lambda$= 789.4 nm) through m = 37 ($\lambda$ = 405.5 nm) appears within a range of 400 nm through 800 nm as the constructive condition. Also, when the optical distance of the layer thickness is 20 $\mu$m, a peak with m = 50 ($\lambda$ = 800 nm) through m = 100 ($\lambda$ = 400 nm) appears as the constructive condition. However, since opposite ends can not be determined as peaks, in actuality, m becomes a range of 51 through 99.

Here, reference will be further made to a case where the optical distance of the layer thickness is about 7.5 $\mu$m. From the result of the Fourier transform in the step of S2 (a peak appears at the 19th pixel), 7.6 $\mu$m (0.4 x 19) is obtained as the optical distance of this layer. The values before and after that value become 7.2 $\mu$m and 8.0 $\mu$m, respectively, so measurements can be made only in increments of 0.4 $\mu$m. On the other hand, let us assume that in the step of S7, a wave number of the smallest maximum value is 2.466 x $10^6$ ($\lambda$ = 405.5 nm). By substituting these values in the above-mentioned expression 1 representing the constructive interference condition, m of 37.03 is obtained. By approximating this value of m to an integer, M = 37 is obtained. In addition, by substituting M thus obtained in the above-mentioned expression 8 in the step of S8, the optical distance of this layer can be obtained more accurately as 7.502 $\mu$m (= 37/(2 x 2.466 x $10^6$)).

Here, measuring precision will be described. The measuring precision indicates positioning accuracy after the respective layers are separated from each other, and the measurement resolution is a spatial distance by which the respective layers can be separated from each other. Since the spectroscope 108 divides the band of 400 nmby 1, 024 pixels, the spectral resolution of the spectroscope 108 is 0.39 nm. An error with respect to m can be removed, so the measuring precision is theoretically represented by the following expression 10. In the above-mentioned condition in which m is approximately equal to 100, themeasuringprecision is about 20 nm. Of course, the smaller the value of m, the more the measuring precision is improved.

$$\delta(nd) = \frac{m\delta\lambda}{2}$$

On the other hand, in case where the optical distances are measured only by discrete Fourier transform, the measuring precision and the measurement resolution are the same and become 400 nm from the above-mentioned expression 4.

Thus, according to the measuring method of the present invention, the measuring precision will be improved to a substantialextent. In other words, since in conventional methods for calculating optical distances in a multilayer structure by using only discrete Fourier transform, only the optical distances denoted by the above-mentioned expression 3 can be represented, and so become discrete values. Thus, in case where actual values exist between discrete values, there occurs so-called spectral leakage in which data disperses in its periphery (i.e., resulting in a waveform with dull peaks in the case of the example of Fig. 3C), and its resolution is decided by the bandwidth of the light source (i.e., the width of the wave number ΔK). On the other hand, in the method of the present invention, a plurality of pieces of data are cut out by means of the band-pass filter, and are then subjected to inverse discrete Fourier transform, whereby a wave-number spectrum of a substantially sine wave form for one layer can be constructed. In other words, leaked spectra are collected to reconstruct a waveform. In this case, it is meant that a sine wave with a period of a non-integer value can be represented by the bandwidth ΔK of the spectroscope. However, it will coincide with the result of the discrete Fourier transform when the bandwidth is a minimum resolution (i.e., 1 pixel). This is because data having leaked into the periphery can not be used. Accordingly, the method of the present invention is effective when the data can be separated with a sufficient bandwidth by means of discrete Fourier transform. In addition, in case where the refractive index depends on the wavelength, more highly accurate measurements can be made because the refractive index corresponding to the wavelength can be used.

Here, there can of course be a case in which a layer construction does not meet a measurement condition. For example, this is a case in which even if the respective layers of a multilayer structure satisfies a condition that can be represented by the above-mentioned expression 3, the state of those layers being added to one another exceeds $nd_{max}$. Even in such a case, it is not that the technique of the present invention can not be used at all though such components are applied to the spectroscope as measurement noise.

In addition, in case where a sweeping light source capable of sweeping wavelength is used as a light source, a photodiode is used as a detector, and a detected signal of the photodiode, after having been amplified by an operational amplifier or the like, is taken into a personal computer through an AD converter. Even in such a case, too, it is possible to use the technique of the present invention.

[Second Embodiment]

In a second embodiment of the present invention, reference will be made to an optical coherence apparatus of a construction which uses a reference mirror, while using the accompanying drawings. Here, the following description will be made mainly about differences from the first embodiment.

<Construction of an Optical System>

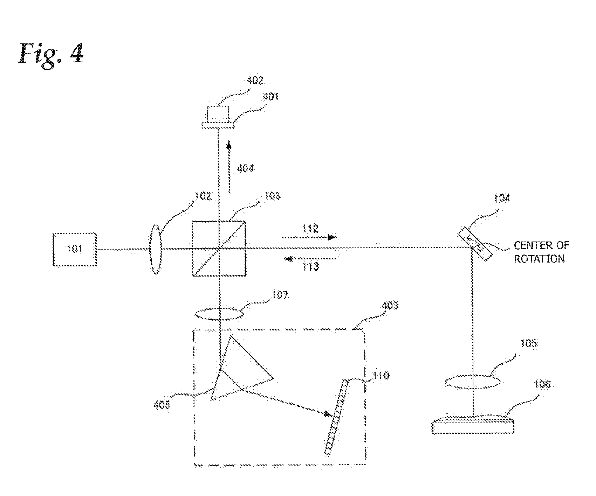

First of all, reference will be made to the construction of the optical coherence apparatus according to this second embodiment by using Fig. 4. The light emitted from a light source 101 is divided into reference light 404 and measurement light 112 by a beam splitter 103. The measurement light is reflected by a sample 106 which is an object to be observed, so that it is returned as return light 113. On the other hand, the reference light is reflected by a reference mirror 401. Here, the reference mirror 401 can adjust an optical path length by a position adjusting mechanism 402. The reference light is combined with the return light by means of the beam splitter 103. Thus, an optical system of the optical coherence apparatus in this second embodiment divides the light from the light source into the measurement light and the reference light, guides the measurement light to the sample 106, also guides the return light thereof to the detection position, and further guides the reference light to the detection position through the reference arm (reference light path).

The combined light reaches the spectroscope 403 through an imaging lens 107. Here, a prism 405 is used for the spectroscope 403. The prism has various merits such as high transmittance, reduced stray light, no overlap of the numbers of order, limited polarization of the emitted light, and so on. On the other hand, there are demerits such as the presence of limitation on materials, the presence of wavelength dependence of dispersion, and so on. Here, a diffraction grating type spectroscope can be used for the spectroscope 403.

Here, reference will be made to the reason for using the reference mirror 401. First of all, the return light from the sample is the light reflected on a surface of the sample, a first interface, a second interface, etc. The electric field of this return light at a wave number k is denoted by $E_s(k)$, and the electric field of the reflected light (reference light) on the reference mirror at the wave number k is denoted by $E_r(k)$. At this time, the optical intensity P(k) of light at the wave number k that is divided to enter an image pickup element 110 becomes a relation as shown by the following expression 11 by using a symbol * which represents a complex conjugate.

$$P(k) \propto E_r^2(k) + E_r(k)E_s^*(k) + E_r^*(k)E_s(k) + E_s^2(k)$$

In case where the reference mirror 401 is not used, the intensity of the light incident on the image pickup element becomes represented by only the fourth term on the right-hand side. When the reflectivity of the sample is low, it becomes impossible to obtain sufficient optical intensity, so light is buried in noise and becomes unable be detected.

On the other hand, in case where the reference mirror 401 is used, all the terms of the above-mentioned expression 11 contribute to the intensity of incident light. Among these, the components related to the interference between the return light from the sample 106 and the reference light from the reference mirror 401 are the second and third terms on the right-hand side. These components become small when $E_s$ is small. With the multilayer structure such as a retina, differences in refractive index between adjacent layers are small, so reflectivity on interfaces between the layers is low and $E_s$ becomes small. Even in such a case, the optical intensity of the light incident on the image pickup element can be raised by increasing the amount of light of the reference light.

In addition, in case where no reference mirror is used, there will be problems such as being unable to know the relative positional relation of the respective layers, being unable to separate the respective layers from one another, and the like. For these reasons, a technique to use a reference mirror can be adopted though the construction of the optical system becomes complicated.

<Optical Distances to the Reference Mirror>

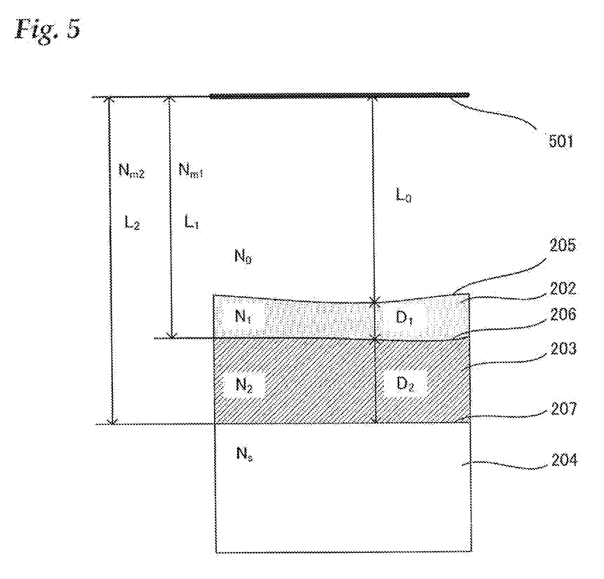

Next, reference will be made to the multilayer structure by using Fig. 5. Here, the influence of multiple reflection is not taken into consideration for the sake of simplicity. In addition, a reference mirror 501 can be considered to be in alignment with the sample, so it is arranged as illustrated in Fig. 5. Here, the optical path difference between the return light and the reference light is denoted as a distance $L_0$ from the reference mirror to a surface of the sample. Also, the optical distance from the reference mirror to the first interface and the optical distance from the reference mirror to the second interface are denoted by $N_{m1}L_1$ and $N_{m2}L_2$, respectively. Here, the sample of the multilayer structure is of a construction having a second layer 203 of a refractive index $N_2$ and a first layer 202 of a refractive index $N_1$ arranged on a substrate 204 of a refractive index $N_s$, and is disposed on a medium of a refractive index $N_0$. The spatial distances of the respective layers are denoted by $D_1$ and $D_2$, respectively.

<Signal Processing>

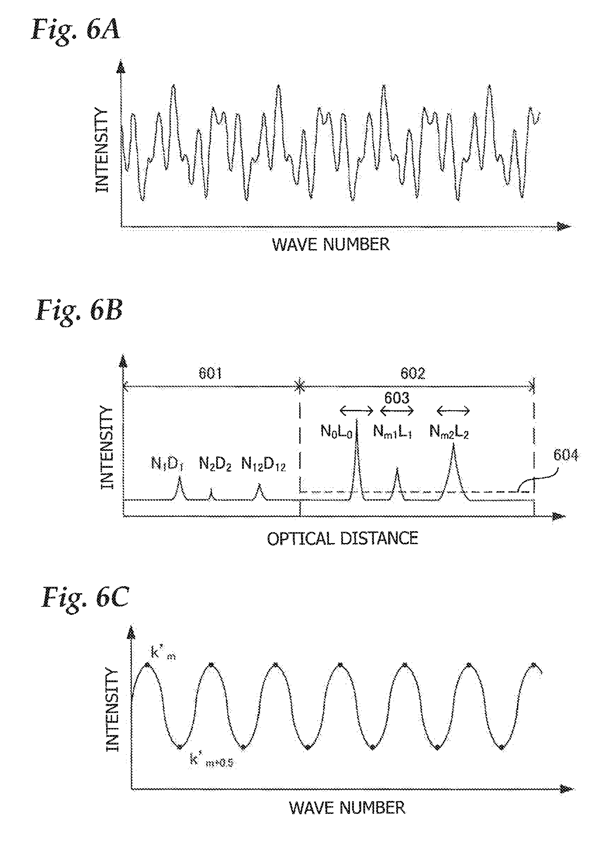

Reference will be made to differences of signal processing steps of this embodiment from those of the first embodiment while taking an example of the construction of Fig. 5. In a step of S1, spectral data is acquired from the spectroscope. The data at this time is diagrammatically illustrated in Fig. 6A.

In a step of S3, filtering is applied to the result of Fourier transform of the spectral data. First of all, the result of the Fourier transform becomes as illustrated in Fig. 6B, in which peaks of the optical distances appear at the positions of $N_0L_0$, $N_{m1}L_1$, and $N_{m2}L_2$. respectively. These optical distances correspond to the optical distances of the sample surface 205, the first interface 206 and the second interface 207, respectively, from the reference mirror 501. At the same time, peaks also appear at $N_1D_1$, $N_2D_2$ and $N_{12}D_{12}$, respectively, due to the interference of the multilayer structure itself, similar to the first embodiment.

Here, it is preferable to set the reference mirror 501 in such a manner that the optical distance $N_0L_0$ between this mirror 501 and the sample surface 205 be longer than the thickness $N_{12}D_{12}$ of the entire multilayer structure. As a result, it is possible to separate a first region 601, in which the interference components of the sample itself appear, and a second region 602, in which the interference components of the sample and the reference mirror appear, from each other.

After one of the peaks obtained as the above result is selected by a filter, the optical distance of the thus selected peak is calculated by a technique similar to that in the above-mentioned first embodiment. In other words, in a step of S4, a result as illustrated in Fig. 6C is obtained by performing inverse Fourier transform on the selected peak.

Then, in a step of S6 (corresponding to a seventh step of the present invention), an order of interference m is calculated from the above-mentioned interference condition expression 1 by specifying a wave number from the result of Fig. 6C. In a step of S7, the order of interference m thus obtained is rounded off to an integer, and the optical distance is then calculated by substituting the integer in the interference condition expression.

Here, in case the reference mirror is used, the distances of the respective layers are in the following relations.

$$N_{m1}L_1 = N_0L_0 + N_1D_1$$

Accordingly, the spatial distance of the first layer becomes as shown in the following expression 13.

$$D_1 = \frac{N_{m1}L_1 - N_0L_0}{N_1}$$

Similarly, the spatial distance of the second layer becomes as shown in the following expression 14.

$$D_2 = \frac{N_{m2}L_2 - N_{m1}L_1}{N_2}$$

By extending this to a (i+1)-th layer, the following expression 15 will be obtained

$$D_{i+1} = \frac{N_{mi+1}L_{i+1} - N_{mi}L_i}{N_{i+1}}$$

In other words, the spatial distance of each layer is obtained by subtracting an optical distance ($N_{mi}L_i$) to an interface thereof at a side near the reference mirror from an optical distance ($N_{mi+1}L_{i+1}$) to an interface thereof at a side far from the reference mirror, and by dividing it by a refractive index ($N_{i+1}$) between both the interfaces.

If these spatial distances are imaged, a tomographic image of the spatial distances based on Fig. 5 can be obtained. Here, a conventional tomographic image is a tomographic image of the optical distances.

[Third Embodiment]

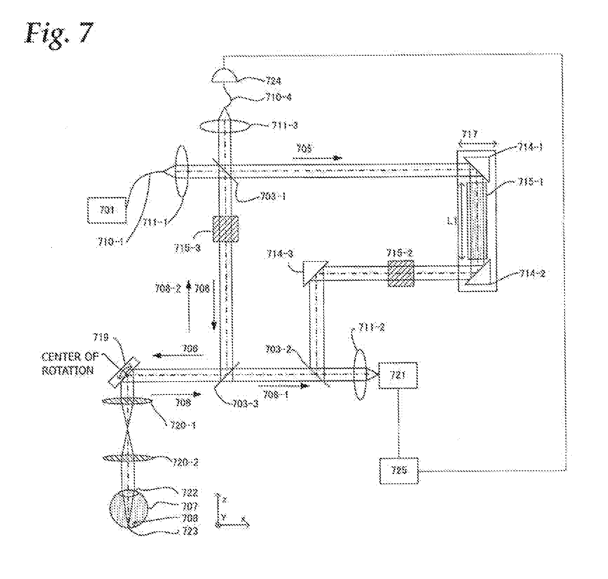

In a third embodiment of the present invention, reference will be made to an optical system in an ophthalmic optical coherence apparatus to which the present invention is applied, while using Fig. 7. The system has a basic construction of the type using the reference mirror of the second embodiment.

<Construction of an Optical System>

Fig. 7 illustrates constructing a Mach-Zehnder interference system as a whole. The light emitted from a light source 701 is divided into reference light 705 and measurement light 706 by means of a beam splitter 703-1. The measurement light 706, after being returned, through reflection or scattering, as return light 708 by an eye 707 which is an object to be observed, is combined with the reference light 705 by means of a beam splitter 703-2 to enter a spectroscope 721.

First, reference will be made to the surroundings of the light source 701. The light source 701 is an SLD (Super Luminescent Diode) that is a typical low coherent light source. The light source 701 has a wavelength of 830 nm, and a bandwidth of 50 nm. Here, the bandwidth is an important parameter because it influences the resolution in the direction of an optical axis of a tomographic image to be obtained. In addition, although the SLD is selected here for the light source, any kind of light source can be used which need only be able to emit low coherent light, and an ASE (Amplified Spontaneous Emission) or the like can be used. Also, in view of the fact that an eye is measured, near infrared light is suitable for the wavelength to be used. Further, it is desirable that the wavelength be as short as possible because it influences the resolution in the horizontal direction of a tomographic image to be obtained. Here, a wavelength of 830 nm is used. Of course, other wavelengths can be selected depending upon a measurement portion of an object to be observed. The light emitted from the light source 701 is guided to a lens 711-1 through a single-mode fiber 710-1.

Next, reference will be made to the optical path of the reference light 705. The reference light 705 divided by the beam splitter 703-1 enters in succession mirrors 714-1 through 714-3, by which it is changed in its travel direction to enter the spectroscope 721 through the beam splitter 703-2. Here, reference numerals 715-1 and 715-2 denote dispersion compensation glasses. The dispersion compensation glass 715-1 compensates for dispersion of the measurement light 706 with respect to the reference light 705 when the measurement light 706 makes a round trip to the eye 707. In other words, it is desirable that the length L1 of the dispersion compensation glass 715-1 be equal to twice the depth of a general eye. Here, the length L1 is set to 46 mm which is twice 23 mm which is assumed to be the diameter of an average eyeball of Japanese. Further, an electric stage 717 is movable in a direction indicated by an arrow, so that it can adjust and control the optical path length of the reference light 705. The dispersion compensation glass 715-2 serves for the purpose of compensating for the dispersion of lenses 720-1 and 720-2 used to scan the eye 707.

Reference will be made to the optical path of the measurement light 706. The measurement light 706 divided by the beam splitter 703-1 passes through a dispersion compensation glass 715-3, and is reflected by a beam splitter 703-3. Then, the measurement light 706 is incident on a mirror of an XY scanner 719. The XY scanner 719 raster-scans a retina 723 in a direction perpendicular to the optical axis of the measurement light 706. In addition, the center of the measurement light 706 is adjusted so as to coincide with the center of rotation of the mirror of the XY scanner 719. The lenses 720-1 and 720-2 together constitute an optical system for scanning the retina 723, and have a role to cause the measurement light 706 to scan the retina 723 with a location in the vicinity of a cornea 722 being placed as a fulcrum. Here, the focal distances of the lenses 720-1 and 720-2 are 50 mm and 50 mm, respectively. When the measurement light 706 enters the eye 707, it is reflected or scattered from the retina 723 as the return light 708. Further, the return light 708 is divided into return light 708-1 and return light 708-2 by means of the beam splitter 703-3, so that the one return light 708-1 is guided to the spectroscope 721, and the other return light 708-2 passes through the beam splitter 703-1 to be guided to a detector 724. An output signal of the detector 724 is electrically taken into a computer 725, similar to a interference signal, so that the intensity of the return light 708-2 can be recorded and displayed. In addition, the signal obtained by the detector 724 is an intensity signal of the return light 708-2 due to the reflection or scattering on the retina 723, and it does not have any depth resolution. For example, an APD (Avalanche Photo Diode), which is a high-speed and high-sensitivity sensor, is used as the detector 724.

<Examples of Numerical Values>

Here, a description will be given by using numerical values. The object to be observed is the eye, and the physical size and the structure thereof do not change so much depending on individual persons. However, for the sake of simplicity, the structure of the eye is assumed as follows. First, the spatial distance of the entire retina is 560 $\mu$m, and is composed of seven layers, and the spatial distance of each layer is 80 $\mu$m. In addition, the refractive index of each layer is 1.5 (although no reflection will occur on each interface between adjacent layers unless the refractive indexes of the layers are different from each other, all the refractive indexes are herein set to the same value for simplifying the calculation of numerical values). Accordingly, the optical distance of each layer is 120 $\mu$m, and the optical distance of the entire retina is 840 $\mu$m. The position of the mirror is set such that the spatial distance $L_0$ becomes 400 $\mu$m ($L_0 = 400$ $\mu$m) and the optical distance thereof is 600 $\mu$m.

When all the spectrum of 805 nm ~ 855 nm is used, a minimum distance $nd_{min}$ that can be measured is 6.9 $\mu$m. Also, when N = 1, 024, a maximum distance $nd_{max}$ that can be measured is 3 mm (optical distance). If the interference spectrum is subjected to discrete Fourier transform, peaks stand out in the vicinity of 87, 104, 122, 139, 157, 174, 191, and 209 pixels because of 6.9 $\mu$m per pixel. These peaks are apart from one another, so they can be separated from one another. When the optical distance is 600 $\mu$m, i.e., the shortest, the constructive condition is m = 1,404 1,490, whereas when the optical distance is 1,440 $\mu$m, i.e., the longest, the constructive condition is m = 3,369 ~ 3,577. On the other hand, in order to divide 50 nm into 1, 024 parts, the resolution of the spectroscope is 0.049 nm. When m = 4,000, the measuring precision will be theoretically about 100 nm, though the condition changes depending upon the wavelength to be calculated. Since the measuring precision according to the discrete Fourier transform is 6.9 $\mu$m, it can be said that the measuring precision is improved to a significant extent.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions. A tomographic image measuring method according to the present invention includes a first step of calculating information corresponding to an optical distance of each layer thickness from a wave-number spectrum, a second step of separating and extracting information of each layer from the information corresponding to the optical distance of each layer thickness, a third step of reconverting information of each layer into a wave-number spectrum, respectively, a fourth step of obtaining a interference wave number from the result of the third step, a fifth step of calculating an order of interference from the interference wave number and the optical distance of each layer; and a sixth step of calculating the optical distance of each layer by making use of the fact that the order of interference is an integer. Thus, when discrete Fourier transform is used, the measuring precision of layers is improved.

**Claims**

**1.** A multilayer structure measuring method in an apparatus which includes:

a light source;

an optical system that guides light from said light source to an object of a multilayer structure to be observed, and at the same time guides return light from said object to be observed to a detection position;

a spectroscope that is disposed at said detection position for detecting a wave-number spectrum of the light incident thereon; and

an analysis unit that measures the multilayer structure of said object to be observed from the detected wave-number spectrum;

said method comprising:

a first step of calculating information corresponding to an optical distance of each layer thickness from said wave-number spectrum;

a second step of separating and extracting information corresponding to an optical distance of each layer from the information corresponding to said optical distance of each layer thickness;

a third step of reconverting information corresponding to said optical distance of each layer into a wave-number spectrum, respectively;

a fourth step of obtaining a wave number or wavelength at which interference occurs from the result of said third step;

a fifth step of calculating an order of interference from said wave number or wavelength obtained in said fourth step and said optical distance of each layer; and

a sixth step of approximating said order of interference obtained in said fifth step to a nearest integer, and calculating the optical distance of each layer from said order of interference thus approximated and said wave number or wavelength at which interference occurs.

2. The multilayer structure measuring method as set forth in claim 1, wherein said first step is a step of applying Fourier transform to said wave-number spectrum.

3. The multilayer structure measuring method as set forth in claim 1 or 2, wherein said second step is to extract information corresponding to the optical distance of each layer by means of a band-pass filter, and a range of extraction for each layer is variable.

4. The multilayer structure measuring method as set forth in any one of claims 1 to 3, wherein said third step is a step to apply inverse Fourier transform to information corresponding to the optical distance.

5. The multilayer structure measuring method as set forth in any one of claims 1 to 4, wherein in said fifth step, said order of interference is calculated by using the fact that twice the optical distance of each layer is an integral multiple of the wavelength.

6. The multilayer structure measuring method as set forth in any one of claims 1 to 5, wherein in said fifth step, said order of interference is calculated by using the fact that twice the optical distance of each layer is a half-integral multiple of the wavelength.

7. The multilayer structure measuring method as set forth in any one of claims 1 to 6, wherein in said fifth and sixth steps, an optical distance which takes a peak in the result of said first step is used as the optical distance of each layer.

8. The multilayer structure measuring method as set forth in any one of claims 1 to 7, further comprising:

a step of obtaining from the result of said third step a plurality of wave numbers or wavelengths at which interference occur, and calculating the optical distance of each layer from said wave numbers or wavelengths thus obtained and a interference condition;

wherein in said fifth and sixth steps, the optical distance of each layer calculated in said step of obtaining from the result of said third step a plurality of wave numbers or wavelengths is used for the calculation of said order of interference.

9. The multilayer structure measuring method as set forth in any one of claims 1 to 8, further comprising:

a seventh step of converting the optical distance of each layer into a spatial distance after said sixth step.

10. The multilayer structure measuring method as set forth in claim 1, wherein said optical system of said apparatus includes:

a unit that divides the light from said light source into measurement light and reference light;
a unit that guides said measurement light to said object to be observed, and at the same time guides return light from said object to be observed to said detection position; and
a unit that guides said reference light to said detection position through a reference arm;
wherein said spectroscope detects a wave-number spectrum of combined light of said return light and said reference light; and
said analysis unit measures the multilayer structure of said object to be observed based on the wave-number spectrum of said combined light.

11. The multilayer structure measuring method as set forth in claim 10, wherein an optical distance of an optical path difference between said reference light and the return light reflected on a surface of said object to be observed is longer than an optical distance of the entire multilayer structure of said object to be observed.

12. The multilayer structure measuring method as set forth in claim 10 or 11, wherein said unit that guides said reference light to said detection position includes a reference mirror that reflects said reference light;
said method further comprises a seventh step of converting the optical distance of each layer into a spatial distance after said sixth step; and
in said seventh step, said spatial distance is calculated by subtracting an optical distance to an interface at a side near said reference mirror from an optical distance to an interface at a side far from said reference mirror, and by dividing it by a refractive index between both said interfaces.

13. A multilayer structure measuring apparatus for measuring a multilayer structure of an object to be observed, said apparatus comprising:

a light source;
an optical system that guides light from said light source to an object of a multilayer structure to be observed, and at the same time guides return light from said object to be observed to a detection position;
a spectroscope that is disposed at said detection position for detecting a wave-number spectrum of the light incident thereon; and
an analysis unit that measures the multilayer structure of said object to be observed from the detected wave-number spectrum;
wherein said analysis unit executes:

a first step of calculating information corresponding to an optical distance of each layer thickness from said wave-number spectrum;
a second step of separating and extracting information corresponding to an optical distance of each layer from the information corresponding to said optical distance of each layer thickness;
a third step of reconverting information corresponding to said optical distance of each layer into a wave-number spectrum, respectively;
a fourth step of obtaining a wave number or wavelength at which interference occurs from the result of said third step;
a fifth step of calculating an order of interference from said wave number or wavelength obtained in said fourth step and said optical distance of each layer; and
a sixth step of approximating said order of interference obtained in said fifth step to a nearest integer, and calculating the optical distance of each layer from said order of interference thus approximated and said wave number or wavelength at which interference occurs.

14. The multilayer structure measuring method as set forth in claim 13, wherein said optical system of said apparatus includes:

a unit that divides the light from said light source into measurement light and reference light;
a unit that guides said measurement light to said object to be observed, and at the same time guides return light from said object to be observed to said detection position; and
a unit that guides said reference light to said detection position through a reference arm;
wherein said spectroscope detects a wave-number spectrum of combined light of said return light and said reference light; and

said analysis unit measures the multilayer structure of said object to be observed based on the wave-number spectrum of said combined light.

*Fig. 1*

*Fig. 2*

*Fig. 3A*

```
              ╭─────────────────────╮
              │        START        │
              │  SIGNAL PROCESSING  │
              ╰─────────────────────╯
                         │
         ┌───────────────┤
         │               ▼
   S1    │    ┌─────────────────────────┐
         │    │  ACQUIRE WAVE NUMBER     │
         │    │       SPECTRUM          │
         │    └─────────────────────────┘
         │               │
         │               ▼
   S2    │    ┌─────────────────────────┐
         │    │  PERFORM FOURIER TRANSFORM │
         │    └─────────────────────────┘
         │               │
         │               ▼◄─────────────────┐
   S3    │    ┌─────────────────────────┐   │
         │    │     EXTRACT ONE PEAK    │   │
         │    └─────────────────────────┘   │
         │               │                  │
         │               ▼                  │
   S4    │    ┌─────────────────────────┐   │
         │    │    PERFORM INVERSE      │   │
         │    │   FOURIER TRANSFORM     │   │
         │    └─────────────────────────┘   │
         │               │                  │
         │               ▼                  │
   S5    │    ╱─────────────────────────╲   │ NO
         │    ╲  PROCESSING OF ALL PEAKS ╱───┘
         │     ╲    COMPLEATED ?       ╱
         │               │
         │              YES
         │               ▼
   S6    │    ┌─────────────────────────┐
         │    │  SPECIFY INTERFERENCE   │
         │    │ WAVE NUMBER (WAVELENGTH)│
         │    └─────────────────────────┘
         │               │
         │               ▼
   S7    │    ┌─────────────────────────┐
         │    │   CALCULATE ORDER OF    │
         │    │    INTERFERENCE m       │
         │    └─────────────────────────┘
         │               │
         │               ▼
   S8    │    ┌─────────────────────────────┐
         │    │ CALCULATE OPTICAL DISTANCE  │
         │    │ OF LAYER THICKNESS BY USING m│
         │    │  APPROXIMATED TO AN INTEGER │
         │    └─────────────────────────────┘
         │               │
         │               ▼
   S9    │    ╱─────────────────────────╲
         └────╲  MEASURING IN ALL REGIONS╱
          NO   ╲    COMPLETED ?         ╱
                         │
                        YES
                         ▼
              ╭─────────────────────╮
              │        END          │
              ╰─────────────────────╯
```

*Fig. 3B*

*Fig. 3C*

*Fig. 3D*

*Fig. 4*

CENTER OF
ROTATION

*Fig. 5*

*Fig. 6A*

*Fig. 6B*

*Fig. 6C*

Fig. 7

Europäisches
Patentamt

European
Patent Office

Office européen
des brevets

**EUROPEAN SEARCH REPORT**

Application Number

EP 09 16 3477

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2004/080761 A1 (DU-NOUR OFER [IL] ET AL) 29 April 2004 (2004-04-29) * paragraph [0101] - paragraph [0188]; figures 1-5 * | 1-9,13 | INV. G01B9/02 G01B11/06 A61B3/10 |
| Y | SEMI, 3081 ZANKER ROAD, SAN JOSE, CA 95134, USA, November 2003 (2003-11), XP040454207 * page 1 - page 4 * | 1-14 | |
| Y | KIM D ET AL: "Measurement of the thickness profile of a transparent thin film deposited upon a pattern structure with an acousto-optic tunable filter" 1 November 2002 (2002-11-01), OPTICS LETTERS 20021101 OPTICAL SOCIETY OF AMERICA US, VOL. 27, NR. 21, PAGE(S) 1893 - 1895 , XP002547752 * page 1893 - page 1894 * | 1-6, 10-14 | |
| Y | WO 2006/125131 A (ZYGO CORP [US]; DE GROOT PETER [US]) 23 November 2006 (2006-11-23) * page 1 - page 3; figures 1-4,13A,13B * * page 11 - page 13 * * page 15 - pages 31,38 * * page 48 - page 50 * | 1,7, 10-14 | TECHNICAL FIELDS SEARCHED (IPC) G01B A61B |

The present search report has been drawn up for all claims

| Place of search | Date of completion of the search | Examiner |
|---|---|---|
| Munich | 28 September 2009 | Stanciu, C |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

**ANNEX TO THE EUROPEAN SEARCH REPORT
ON EUROPEAN PATENT APPLICATION NO.**

EP 09 16 3477

This annex lists the patent family members relating to the patent documents cited in the above-mentioned European search report.
The members are as contained in the European Patent Office EDP file on
The European Patent Office is in no way liable for these particulars which are merely given for the purpose of information.

28-09-2009

| Patent document cited in search report | | Publication date | Patent family member(s) | | Publication date |
|---|---|---|---|---|---|
| US 2004080761 | A1 | 29-04-2004 | AU | 2003276649 A1 | 13-05-2004 |
| | | | CN | 1732372 A | 08-02-2006 |
| | | | EP | 1556668 A2 | 27-07-2005 |
| | | | WO | 2004038321 A2 | 06-05-2004 |
| | | | JP | 2006504092 T | 02-02-2006 |
| | | | KR | 20050083844 A | 26-08-2005 |
| WO 2006125131 | A | 23-11-2006 | EP | 1883781 A2 | 06-02-2008 |
| | | | JP | 2008545957 T | 18-12-2008 |
| | | | KR | 20080015018 A | 15-02-2008 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H11325849 B